# FASCICULE DE BREVET EUROPEEN

(11) **EP 2 521 906 B1**
(45) Date de publication et mention de la délivrance du brevet: **23.10.2013**
(21) Numéro de dépôt: 11700246.9
(22) Date de dépôt: 07.01.2011
(51) Int. Cl.: G01N 33/38, G01N 25/72

(54) **PROCEDE DE MESURE DE LA CORROSION DANS UN OUVRAGE EN BETON**
VERFAHREN ZUR MESSUNG VON KORROSION BEI EINEM BETONGEBÄUDE
METHOD FOR MEASURING CORROSION IN A CONCRETE BUILDING

(30) Priorité: 08.01.2010 FR 1050100
(43) Date de publication de la demande: 14.11.2012
(73) Titulaire: Bouygues Travaux Publics, 78280 Guyancourt (FR)
(72) Inventeur: BARBERON, Fabien, F-92800 Puteaux (FR); GEGOUT, Philippe, F-78610 Les Breviaires (FR); LOPEZ-RIOS, Julien, F-38330 St Ismier (FR)
(74) Mandataire: Regimbeau
(86) Numéro de dépôt international: PCT/EP2011/050162
(87) Numéro de publication internationale: WO 2011/083142

(56) Documents cités:
- JP-A- 10 318 956
- JP-A- 2003 139 731
- JP-A- 2006 337 232
- BRACHELET, F; DU, T; DEFER, D; ANTCZAK, E: "Detection of poor filling in prestressed beams specimen byinductive thermography and transfer function analysis" In: "NDTCE09 - 7th International Symposiumon Non Destructive Testing in Civil Engineering", 30 juin 2009 (2009-06-30), NDTCE09, Nantes, XP002596640, le document en entier

## Description

### DOMAINE DE L'INVENTION

La présente invention concerne un procédé de mesure de la corrosion d'un élément métallique dans un ouvrage en béton.

### ARRIERE PLAN DE L'INVENTION

Le contrôle de la corrosion d'éléments métalliques, tels que des armatures, dans des ouvrages en béton armé, est une problématique importante dans le domaine du génie civil puisqu'il vise à s'assurer de la sécurité desdits ouvrages.

Des méthodes de contrôle non destructif ont été développées à cet effet.

Ainsi, le document WO 2009/007395 décrit des capteurs permettant de contrôler la corrosion d'armatures métalliques dans des ouvrages en béton.

Ces capteurs, qui sont noyés dans le volume de béton, sont constitués d'un substrat résistant à la corrosion et d'un élément métallique, par exemple une couche mince, dont l'état de surface est agencé pour initier et guider la propagation de la corrosion, afin d'éviter une corrosion aléatoire des différents capteurs.

La corrosion des capteurs à différentes profondeurs est indicative de la vitesse de progression du front de corrosion.

Ces capteurs utilisent la propriété des ondes électromagnétiques de ne pénétrer dans les métaux que sur une très mince profondeur (plus petite que l'épaisseur de la lame utilisée comme capteur) et de pénétrer par contre sans difficulté dans les métaux oxydés et plus généralement dans les matériaux ayant un caractère diélectrique comme le béton.

L'outil choisi pour déceler cette propriété a été le radar (GHz).

En effet, l'onde radar est réfléchie par les capteurs métalliques non oxydés et passe à travers les capteurs oxydés. On a donc une signature radar uniquement pour les capteurs non oxydés.

Cependant, la détection par radar comporte certains inconvénients.

En particulier, il est difficile d'effectuer des mesures à faible profondeur (c'est-à-dire dans les 2 ou 3 premiers centimètres sous la surface du béton).

Par ailleurs, l'interprétation des mesures est particulièrement délicate.

Enfin, le dispositif est coûteux et l'instrument de mesure présente un poids et un volume élevés, ce qui rend malaisées des mesures sur site.

L'inspection de béton armé par thermographie est connue de JP2006337232, JP2003139731 et JP10318956.

Or il serait souhaitable de pouvoir observer les capteurs et suivre leur corrosion en continu.

Le but de la présente invention est donc de déterminer un procédé pour observer l'oxydation des capteurs qui permette d'obtenir des images avec une meilleure résolution, être moins onéreux et de mise en oeuvre aisée.

Un autre but de l'invention est de permettre de mesurer la corrosion même à une très faible profondeur sous la surface du béton.

### BREVE DESCRIPTION DE L'INVENTION

Conformément à l'invention, il est proposé un procédé de contrôle de la corrosion d'un ouvrage en béton, ledit ouvrage comprenant au moins un capteur comportant un élément métallique situé à une profondeur déterminée entre la surface du béton et le premier lit d'armatures, ledit procédé étant caractérisé en ce qu'il comprend, pour ledit capteur, la mesure de la corrosion dudit élément métallique par la mise en oeuvre de :
- un chauffage par induction dudit élément métallique, par application d'une excitation magnétique au-dessus de la surface de l'ouvrage,
- la réalisation d'au moins une image thermographique de la surface de l'ouvrage, la déduction, à partir de l'image thermographique, du degré de corrosion de l'élément métallique.

Selon un mode possible de mise en oeuvre du procédé, on réalise plusieurs images thermographiques successives après l'arrêt de l'excitation magnétique, de manière à mesurer la variation de température dudit élément métallique au cours du temps.

Selon un autre mode possible de mise en oeuvre du procédé, dans le cas où l'ouvrage comporte plusieurs éléments métalliques susceptibles de présenter des niveaux de corrosion différents, on réalise plusieurs images thermographiques successives après l'arrêt de l'excitation magnétique, de manière à discriminer, par leur température, au moins deux éléments métalliques visibles sur lesdites images.

De préférence, l'excitation magnétique est appliquée avec un champ magnétique d'intensité supérieure à 0,1 mT, une fréquence comprise entre 10 kHZ et 1 MHz et à une distance comprise entre 0,1 cm et 10 cm par rapport à la surface de l'ouvrage.

De manière particulièrement avantageuse, le capteur est disposé à une profondeur comprise entre 0,1 et 10 cm sous la surface du béton.

Selon un mode préféré de réalisation, le capteur est constitué d'une feuille métallique dont l'épaisseur est comprise entre 20 et 500 micromètres, et qui est sensiblement parallèle à la surface du béton.

Eventuellement, l'élément métallique comprend un état de surface adapté de manière à contrôler la localisation de l'initiation de la corrosion et, le cas échéant, la propagation de celle-ci.

De préférence, les dimensions de l'élément métallique et la durée de l'excitation magnétique sont choisies pour conduire à une élévation de sa température d'au moins 2°C.

Un autre objet de l'invention concerne enfin un procédé de fabrication d'un ouvrage en béton, dans lequel on met en place dans le béton au moins un capteur comprenant un élément métallique et on met en oeuvre le procédé de contrôle de la corrosion qui vient d'être décrit.

### BREVE DESCRIPTION DES DESSINS

D'autres caractéristiques et avantages de l'invention ressortiront de la description détaillée qui va suivre, en référence aux dessins annexés sur lesquels :
- la figure 1 est un schéma illustrant le principe de l'invention,
- la figure 2 illustre l'agencement de capteurs dans un bloc de béton utilisé dans le premier exemple expérimental,
- les figures 2A à 2D illustrent les images thermographiques de la surface du bloc de béton de la figure 2 obtenues à différents instants après l'arrêt de l'excitation magnétique,
- la figure 3 illustre l'agencement de capteurs dans un bloc de béton utilisé dans le deuxième exemple expérimental,
- les figures 3A et 3B illustrent les images thermographiques (a) de la surface et (b) du côté du bloc de béton de la figure 3 obtenues à différents instants après l'arrêt de l'excitation magnétique,
- les figures 4A et 4B sont des graphiques illustrant respectivement la dépendance de la température atteinte par un capteur en fonction de l'épaisseur de béton sous laquelle il est situé, ainsi que la température atteinte par des capteurs de différentes tailles et pour différentes durées d'excitation en fonction de l'épaisseur de béton recouvrant lesdits capteurs,
- la figure 5 illustre l'agencement d'un capteur et d'une barre métallique dans un bloc de béton utilisé dans le troisième exemple expérimental,
- la figure 5A illustre les images thermographiques (a) de la surface et (b) du côté du bloc de béton de la figure 5 obtenues à différents instants après l'arrêt de l'excitation magnétique.

### DESCRIPTION DETAILLEE DE L'INVENTION

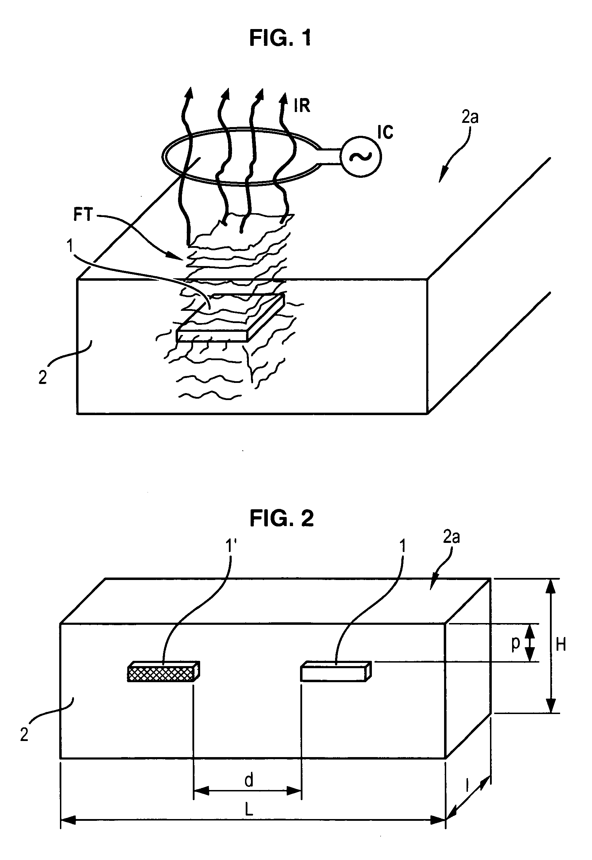

La figure 1 explique le principe de l'invention.

Au moins un capteur 1 est noyé dans un volume de béton 2.

On positionne, à proximité de la surface 2a du volume de béton 2, et en vis-à-vis du capteur 1, une bobine dans laquelle circule un courant alternatif IC apte à générer un courant induit dans le capteur 1.

Un capteur intact, c'est-à-dire peu ou pas oxydé, est chauffé par induction, tandis qu'un capteur oxydé ne l'est pas.

Le capteur 1 chauffé génère un flux thermique FT à travers le volume de béton qui augmente la température à la surface 2a, ce qui se traduit par une augmentation conséquente de l'émission de photons infrarouge IR dans une zone dont la forme et la surface correspondent sensiblement à celles du capteur.

Une image thermique obtenue au moyen d'une caméra infrarouge permet de visualiser les capteurs chauffés, et d'en déduire leur niveau d'oxydation.

Les récents progrès en thermographie infrarouge permettent de mesurer couramment, avec des caméras non refroidies et à un prix abordable, des variations inférieures à 0,1°C.

### Capteur

De manière générale, le capteur peut être tout objet comprenant en surface au moins un élément métallique, quelle que soit sa forme (plan, cube, sphère, fil, etc.).

Cependant, la sensibilité de la mesure variera d'une forme à une autre.

De manière préférée, on choisira un capteur de forme plane que l'on disposera parallèlement à la surface du béton.

Ainsi, selon un mode de réalisation, le capteur peut se présenter, comme dans le document WO 2009/007395, sous la forme d'un substrat plan en un matériau résistant à la corrosion (par exemple du plastique ou de la céramique), sur lequel est appliqué un élément métallique consistant en une couche métallique présentant un état de surface adapté de façon à contrôler la localisation de l'initiation de la corrosion et, le cas échéant, la progression de celle-ci.

Ladite couche métallique présente généralement une épaisseur comprise entre 20 et 500 µm, l'épaisseur du substrat étant de l'ordre de 100 µm à 1 cm.

Toutefois, le capteur est de préférence constitué uniquement d'un élément métallique (par exemple une feuille de fer), dans la mesure où des composants en d'autres matériaux seraient susceptibles d'absorber le flux thermique émis par l'élément métallique et donc de perturber la mesure.

L'épaisseur dudit élément métallique est de préférence comprise entre 20 et 500 µm, l'homme du métier étant à même de déterminer l'épaisseur optimale en fonction de la sensibilité attendue du capteur. Ainsi, plus l'élément métallique est épais, plus il devra être corrodé pour que la corrosion soit détectable par l'imagerie thermographique : ce type de capteur sera donc avantageux lorsque l'on souhaite vérifier la corrosion de l'ouvrage après une longue durée de vie. En revanche, si l'on souhaite pouvoir contrôler la présence de corrosion à bref délai après la construction de l'ouvrage, on choisira un élément métallique plus fin.

Par ailleurs, la surface de l'élément métallique peut présenter au moins une discontinuité, par exemple une ou plusieurs rayures dont la profondeur est comprise entre 1 et 50 µm et présentant un angle vif avec la surface de la couche métallique.

Cet état de surface particulier permet que la corrosion s'initie au niveau de ces rayures, puis se propage le long de celles-ci.

La fabrication de tels capteurs et les différents modes de réalisation sont décrits de manière détaillée dans le document WO 2009/007395 et ne seront donc pas décrits à nouveau en détail ici.

Cependant, l'homme du métier pourra choisir de ne pas procurer une telle discontinuité à la surface du capteur.

### Système de chauffage

Le système de chauffage par induction comprend typiquement un générateur de courant de haute fréquence, une bobine alimentée par ledit générateur et l'élément à chauffer qui est électriquement conducteur (ici, le capteur 1).

Le principe de fonctionnement est basé sur la loi de Lentz, qui permet le transfert par induction de l'énergie électromagnétique d'un circuit à un autre. En effet, une spire ou une bobine parcourue par un courant électrique génère un champ magnétique. Si ce champ magnétique varie périodiquement au voisinage d'un conducteur, des courants de Foucault sont générés dans ledit conducteur qui, en vertu de la loi d'Ohm, produisent de la chaleur. Ceci permet de chauffer le capteur métallique sans contact.

A haute fréquence, les courants ne circulent qu'à la périphérie des conducteurs (effet de peau). On utilise donc de préférence pour la bobine plusieurs fils isolés entre eux, afin de maximiser le volume dans lequel circulent les courants et ainsi optimiser le chauffage.

A titre d'exemple, un prototype a été réalisé avec une bobine de 10 à 15 cm de diamètre, constituée de fils de 2 mm de diamètre, mais l'homme du métier est à même de définir d'autres dimensions de bobine en fonction du champ magnétique qu'il souhaite générer.

De manière avantageuse, la bobine génère un champ magnétique alternatif de 27 kHz de fréquence, qui est assez puissant pour chauffer le capteur 1 enterré sous quelques centimètres de béton.

La valeur efficace du champ magnétique alternatif ainsi généré à 4 cm dans l'air est alors de 50 mT environ.

On peut toutefois choisir une fréquence comprise entre 10 kHz et 1 MHz, en tenant compte du fait que plus on monte en fréquence plus la sensibilité à la corrosion augmente.

La technologie de génération du champ magnétique pourra éventuellement être modifiée en fonction de l'intensité que l'on souhaite obtenir (par exemple, supraconductivité pour des champs magnétiques intenses).

### Thermographie infrarouge

Pour les mesures d'imagerie thermique, on peut utiliser par exemple une caméra thermographique infrarouge T200 commercialisée par FLIR, qui présente une résolution de 200 x 150 pixels, et une sensibilité thermique inférieure à 0,1 °C.

On mesure sur les images les variations spatiales d'intensité de lumière infrarouge.

On peut ainsi procéder à un étalonnage en associant à un degré de corrosion donné une température donnée au bout d'un temps donné après la fin de l'excitation.

### Exemple expérimental n°1 : effet de la corrosion

A titre expérimental, on a pris un bloc de béton fin de 15 cm de longueur L, 6 cm de hauteur H et 4 cm de largeur I.

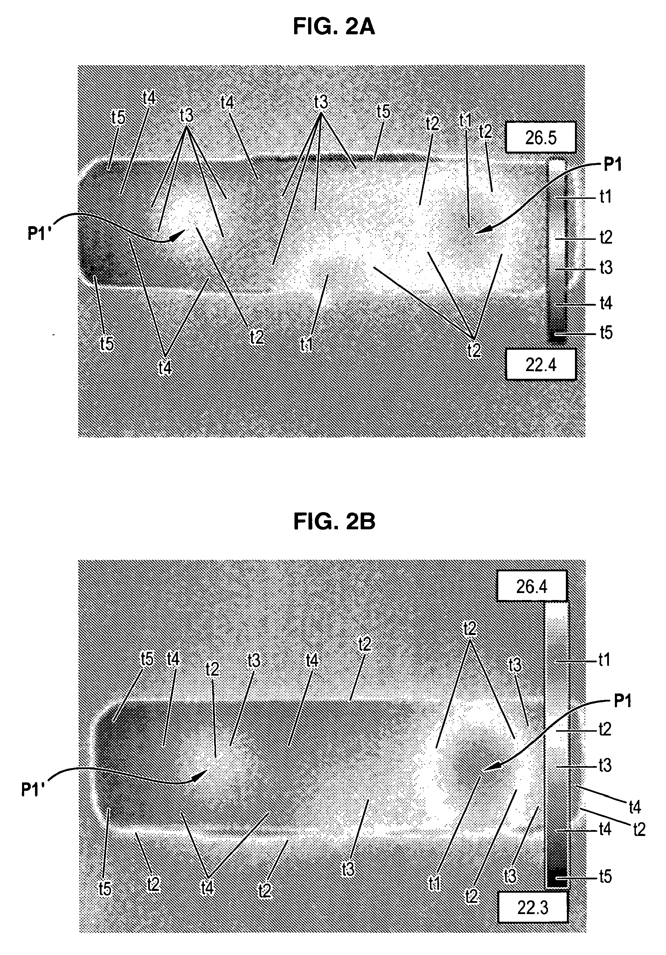

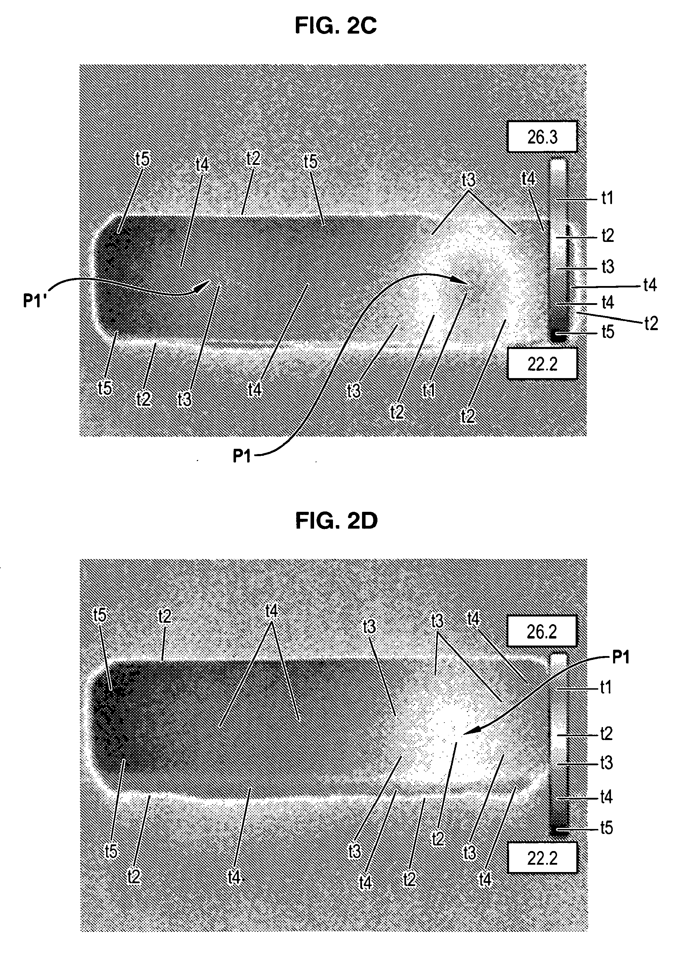

En référence à la figure 2, on a auparavant enterré dans ce bloc 2 deux capteurs 1,1': le capteur 1 est intact, c'est-à-dire non oxydé, tandis que le capteur 1' est partiellement corrodé.

Dans tous les exemples qui suivent, les capteurs sont constitués d'une feuille de fer pur à 99,99% mise en place lors de la coulée du béton.

Les dimensions des capteurs 1, 1' sont : une largeur et une longueur de 2 cm x 2 cm, et une épaisseur de 100 micromètres.

Les deux capteurs sont situés à une profondeur p de 2 cm sous la surface 2a du bloc et sont distants d'une distance d de 7 cm environ.

Le bloc a ensuite été excité par induction électromagnétique pendant 50 secondes au moyen du système décrit plus haut (fréquence de 27 kHz), et des images thermiques de la surface 2a du bloc ont été prises à différents instants après l'arrêt de l'excitation, pendant 5 minutes.

La figure 2A illustre l'image thermographique obtenue juste après l'arrêt de l'excitation.

Sur cette figure comme pour les figures 2B à 2D, 3A, 3B et 4A, on a indiqué à droite de la photographie une échelle indiquant les températures minimale et maximale mesurées, exprimées en °C. Par ailleurs, les zones à iso-température de la surface du bloc ont été repérées par un numéro précédé de la lettre t et référencé sur l'échelle de température.

On observe un point chaud P1, P1' au droit de chacun des capteurs 1, 1' enterrés. (Un troisième point chaud peut être observé dans la partie centrale inférieure : il est dû à la manipulation du bloc 2 par un opérateur et, comme on le verra sur les figures suivantes, il s'estompe très rapidement.)

Par ailleurs, le capteur 1 non corrodé génère un point chaud P1 à une température plus élevée que celle du point chaud P1' généré par le capteur partiellement coloré 1' (soit, respectivement, environ 26,5°C contre 24,5°C).

La figure 2B illustre l'image thermographique obtenue 30 secondes après la fin de l'excitation.

On observe que le point chaud P1 généré par le capteur 1 non corrodé continue d'être très intense, tandis que le point chaud P1' généré par le capteur 1' partiellement corrodé s'estompe. On constate par ailleurs que le point chaud généré par les doigts de l'opérateur a quasiment disparu.

La figure 2C illustre l'image thermographique obtenue 70 secondes après la fin de l'excitation.

On peut constater que le point chaud P1' a pratiquement disparu, tandis que le point chaud P1 conserve son intensité initiale.

Enfin, la figure 2D illustre l'image thermographique obtenue 110 secondes après la fin de l'excitation.

Le point chaud lié au capteur 1' partiellement corrodé a totalement disparu, tandis que le point chaud P1 généré par le capteur 1 non corrodé reste visible, mais avec une température diminuée (environ 24°C).

Au bout de 5 minutes, on ne distingue plus le point chaud P1, la température de surface du bloc 2 étant redevenue uniforme.

### Exemple expérimental n°2 : effet de la profondeur

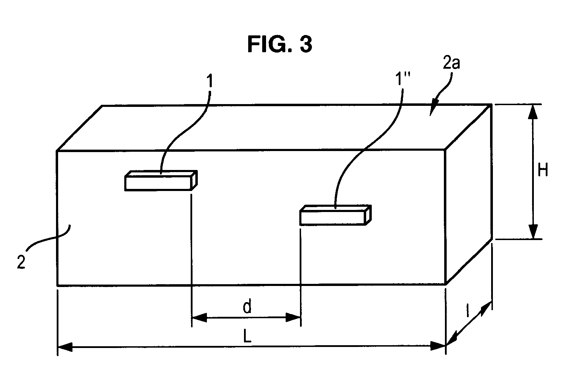

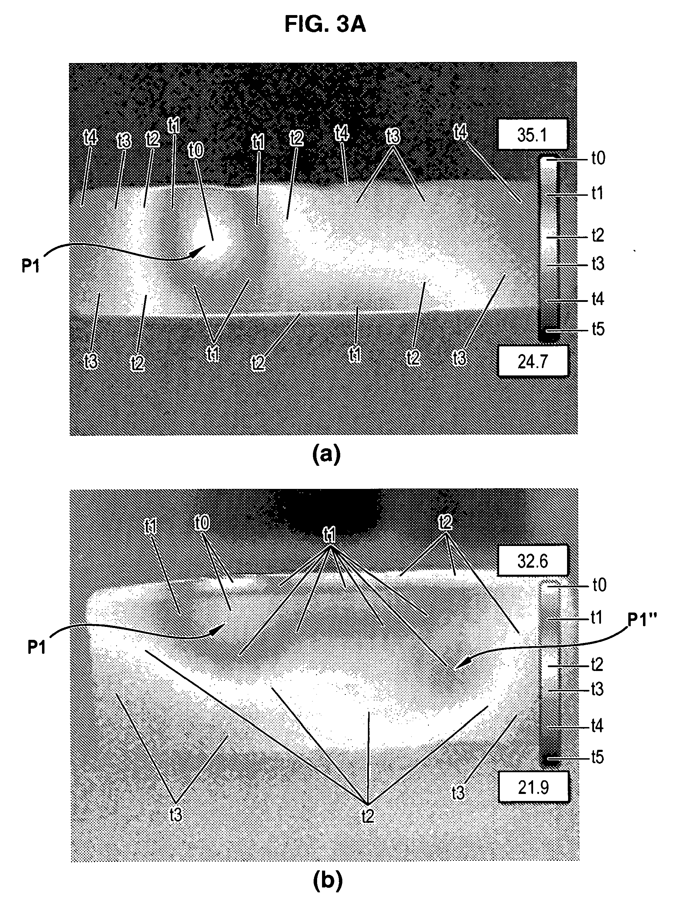

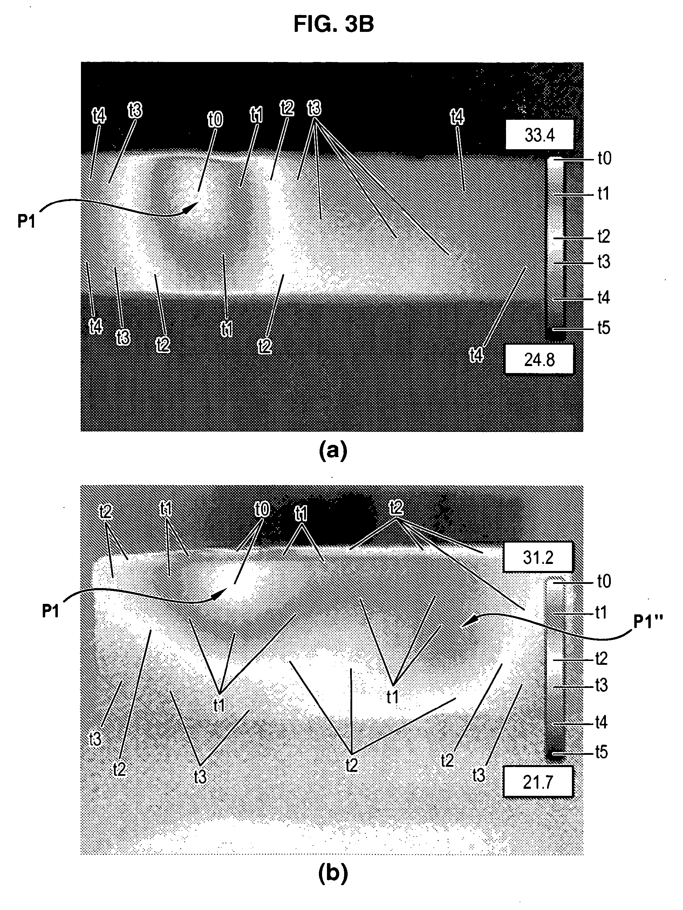

Dans ce deuxième exemple, on fabrique, comme illustré à la figure 3, un bloc de béton 2 de mêmes dimensions que celui de l'exemple précédent, dans lequel on enterre deux capteurs non corrodés 1 et 1", identiques au capteur 1 de l'exemple précédent, situés respectivement à 1,5 et 3 cm de profondeur, et distants de 7 cm environ.

On applique une excitation pendant une durée de 2 minutes environ (de premiers essais avec la même durée que dans l'exemple précédent n'ayant pas permis de mettre en évidence le capteur le plus profond).

La figure 3A illustre en les images thermographiques respectivement de la surface (a) et du côté (b) du bloc de béton 2 juste après l'arrêt de l'excitation.

On observe bien sur la vue (b) de profil les points chauds P1 et P1" générés respectivement par les capteurs 1 et 1".

On peut constater que la température du point chaud P1 généré par le capteur 1 le moins profond est beaucoup plus élevée que celle du point chaud P1" généré par le capteur 1" le plus profond (c'est-à-dire 35°C contre 33°C environ, respectivement).

Ceci est dû au fait que la bobine utilisée génère un champ magnétique qui décroît rapidement avec la distance.

La visualisation du point chaud généré par le capteur 1" le plus profond est moins aisée en vue de dessus (a) que de profil ; en effet, du fait de cette plus grande profondeur, le flux thermique provenant de ce capteur met plus de temps à se propager et arrive de manière atténuée à la surface.

En vue de dessus (a), on observe donc essentiellement le point chaud P1 généré par le capteur 1 le moins profond.

La figure 3B illustre les images thermographiques obtenues 1 minute après l'arrêt de l'excitation.

Compte tenu du fait de l'influence importante de la profondeur du capteur dans l'ouvrage, on détermine ci-après les dimensions du capteur et la durée d'excitation appropriés en fonction de la profondeur du capteur.

A cet effet, on sépare le capteur et le système de chauffage d'une tranche de béton d'épaisseur variable et on relève, après excitation, la température du capteur.

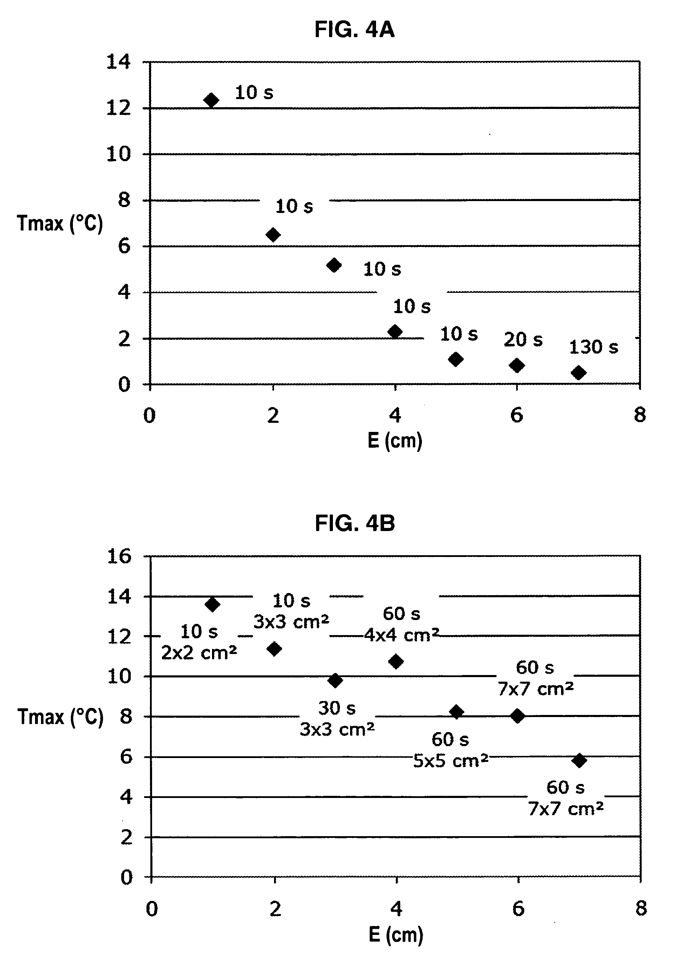

Le graphe de la figure 4A présente l'élévation de température maximale Tmax d'un capteur de 2x2 cm² et de 100 micromètres d'épaisseur en fonction de l'épaisseur E de béton le recouvrant.

La durée de l'excitation (en secondes) est indiquée pour chaque point de mesure.

On observe une nette décroissance de la température atteinte par le capteur avec l'épaisseur de béton.

Ceci implique que l'induction magnétique n'est pas suffisante pour chauffer une aussi petite surface de métal, lorsque l'épaisseur de béton devient importante.

Dans une deuxième étape, on détermine la surface de capteur nécessaire pour obtenir, par exemple, une élévation de température maximale supérieure à 5°C pour avoir une bonne aisance de lecture de la température avec une durée d'excitation inférieure à 60 secondes.

Le graphe de la figure 4B présente les résultats obtenus avec différentes surfaces de capteurs (l'épaisseur étant toujours de 100 micromètres), différentes durées d'excitation et différentes épaisseurs de béton.

On en conclut que, plus la profondeur du capteur est importante, plus la surface de celui-ci et la durée d'excitation doivent être élevées pour que l'élévation de température du capteur soit détectable par la caméra thermographique.

### Exemple expérimental n°3 : influence de la présence d'armatures métalliques

L'influence de la présence d'armatures métalliques, que l'on utilise très couramment dans des ouvrages en béton, a également été évaluée.

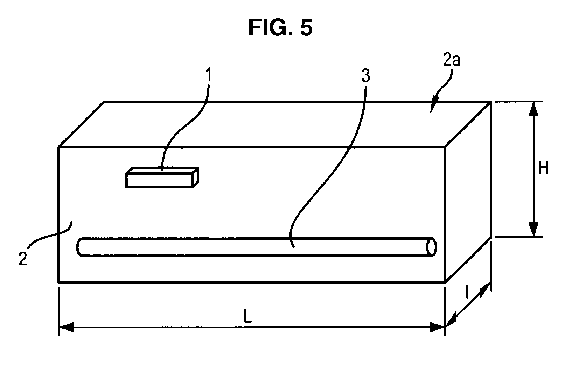

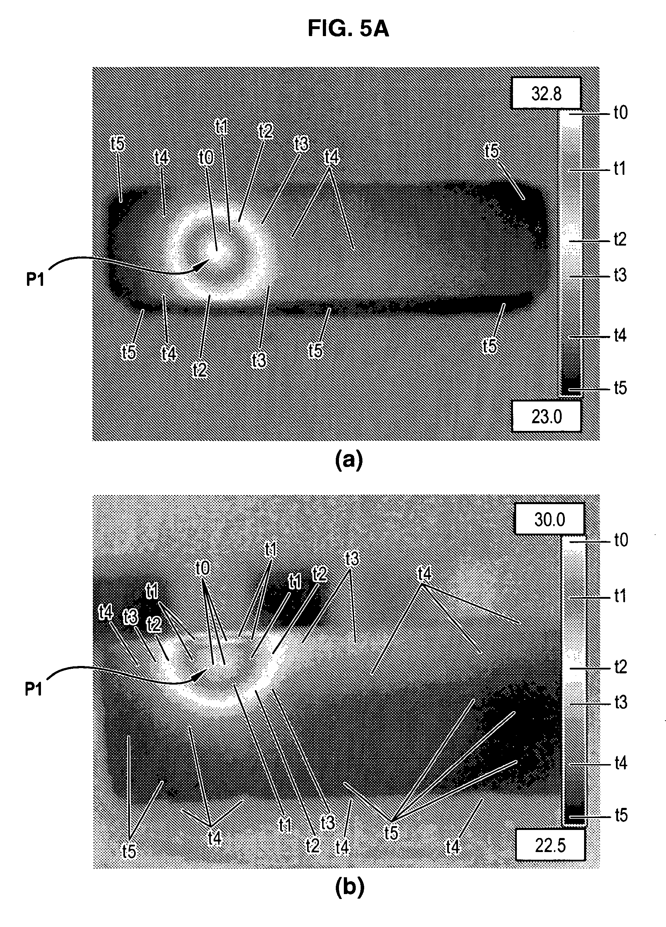

A cet effet, on a construit, comme illustré à la figure 5, un bloc de béton 2 de dimensions identiques à celui des exemples précédents, et dans lequel on a enterré un capteur 1 non corrodé identique au capteur 1 des exemples précédents, à une profondeur de 1 cm, et une barre métallique 3 de 10 mm de diamètre à une profondeur de 4 cm dans le sens de la longueur du bloc 2.

Le bloc a été excité avec le même dispositif que précédemment pendant une durée de 50 secondes.

La figure 5A illustre les images thermographiques (a) de la surface 2a du bloc 2 et (b) du côté.

On constate que l'on observe le point chaud P1 généré par le capteur 1, mais aucun point chaud généré par la barre 3.

L'échauffement du capteur 1 non corrodé est en effet très favorisé par rapport à celui de la barre 3, car seule l'épaisseur de peau chauffe lors de l'excitation par induction magnétique.

Le capteur 1 ayant un volume moins important comparé à sa surface, la puissance thermique distribuée par unité de volume est supérieure, et l'élévation de température est donc plus importante.

### Exemple n°4 : Exemples de paramètres optimisés en conditions réelles

Les paramètres de mesure sont dépendants des appareils de chauffage et de lecture.

Pour déterminer ces paramètres avec les appareils disponibles, un plot en béton a été réalisé dans lequel des capteurs circulaires de différents diamètres, présentant une épaisseur de 100 micromètres et à différentes profondeurs ont été disposés.

Les paramètres du tableau ci-dessous ont ainsi pu être mis en évidence.

Les capteurs ont été chauffés avec un générateur similaire à celui des exemples précédents.

Les résultats ont été obtenus à partir d'images prises par une caméra infrarouge IC 080 V de la société TROTEC, ayant une résolution de 160 x 120 pixels et une sensibilité thermique de 0,1 °C.

Une durée d'excitation de quelques minutes est jugée acceptable pour le contrôle d'un ouvrage.

| Profondeur du capteur (cm) | Diamètre du capteur (cm) | Surface du capteur (cm²) | Temps d'induction (min) |
|---|---|---|---|
| 1 | 2 | 3 | 2 |
| 2 | 6,2 | 30 | 2 |
| 3 | 10 | 80 | 3 |
| 4 | 10 | 80 | 3 |

Dans les constructions de bâtiments et d'ouvrages d'art en béton, les armatures métalliques sont longues et enterrées plus profondément que les capteurs. Par conséquent, la quasi-totalité de l'armature n'est pas excitée par induction ; le reste de l'armature sert alors de radiateur pour dissiper la chaleur, ce qui génère une élévation de température encore plus faible.

La méthode qui vient d'être décrite permet donc de discriminer sans ambiguïté différents niveaux de corrosion de capteurs enterrés dans un ouvrage en béton.

Comme cette méthode provoque préférentiellement un échauffement des éléments conducteurs présentant un ratio surface / volume important (ce qui est le cas des capteurs), la mesure n'est pas perturbée par la présence des habituelles armatures métalliques.

La mesure est également réalisable quelle que soit la profondeur des capteurs (sous réserve d'appliquer une durée d'excitation appropriée, comme on l'a vu plus haut). Notamment, et contrairement à ce qui se produit avec les radars (effet de zone morte), on peut utiliser des capteurs situés très près de la surface du béton.

La profondeur minimale est déterminée en fonction du type d'ouvrage dans lequel seront installés les capteurs. Suivant le type d'ouvrage, cette profondeur peut être comprise entre quelques mm et quelques dizaines de mm.

Pour la fabrication de l'ouvrage dont on souhaite pouvoir contrôler ultérieurement la corrosion par le procédé qui vient d'être décrit, on positionne un ou plusieurs capteurs, de préférence à différentes profondeurs, lors de la coulée du béton.

D'une manière générale, les capteurs sont mis en place entre la surface du béton et le premier lit d'armatures, c'est-à-dire entre 0,1 et 8 à 10 cm suivant le type d'ouvrage.

Les capteurs sont orientés de façon à ce que leur surface la plus importante soit parallèle à la surface du béton.

Il est possible de prévoir un repérage sur ou dans le béton pour identifier l'emplacement et/ou la profondeur des capteurs. Par exemple, une puce peut être installée dans le béton pour indiquer les caractéristiques des différents capteurs présents.

De manière avantageuse, on réalise une calibration de chaque capteur en prenant une ou plusieurs images thermographiques peu de temps après la réalisation de l'ouvrage.

Ces images servent ensuite de référence et pourront être comparées à des images prises ultérieurement, de manière à déterminer si le capteur s'est corrodé, ce qui permet de définir, au fil du temps, l'évolution du front de corrosion dans l'ouvrage.

Un autre avantage de cette méthode est qu'elle permet une analyse directement à partir des images thermographiques, et qu'elle est mise en oeuvre en quelques minutes, avec un appareillage plus léger et moins onéreux que les appareillages existants.

## Revendications

1. Procédé de contrôle de la corrosion d'un ouvrage en béton, ledit ouvrage comprenant au moins un capteur comportant un élément métallique (1, 1', 1"), à une profondeur déterminée entre la surface du béton et le premier lit d'armatures, ledit procédé étant **caractérisé en ce qu'**il comprend, pour ledit capteur, la mesure de la corrosion dudit élément métallique par la mise en oeuvre de :
• un chauffage par induction dudit élément métallique (1, 1', 1"), par application d'une excitation magnétique au-dessus de la surface (2a) de l'ouvrage (2),
• la réalisation d'au moins une image thermographique de la surface (2a) de l'ouvrage (2)
• la déduction, à partir de ladite image thermographique, du degré de corrosion de l'élément métallique (1, 1', 1"').

2. Procédé selon la revendication 1, **caractérisé en ce que** l'on réalise plusieurs images thermographiques successives après l'arrêt de l'excitation magnétique, de manière à mesurer la variation de température dudit élément métallique (1, 1', 1 ") au cours du temps.

3. Procédé selon la revendication 1, **caractérisé en ce que** l'on réalise plusieurs images thermographiques successives après l'arrêt de l'excitation magnétique, de manière à discriminer, par leur température, au moins deux éléments métalliques (1, 1', 1 ") visibles sur lesdites images.

4. Procédé selon l'une des revendications 1 à 3, **caractérisé en ce que** l'excitation magnétique est appliquée avec un champ magnétique d'intensité supérieure à 0,1 mT, une fréquence comprise entre 10 kHZ et 1 MHz et à une distance comprise entre 0,1 cm et 10 cm par rapport à la surface (2a) de l'ouvrage (2).

5. Procédé selon l'une des revendications 1 à 4, **caractérisé en ce que** le capteur est disposé à une profondeur comprise entre 0,1 et 10 cm sous la surface du béton.

6. Procédé selon l'une des revendications 1 à 5, **caractérisé en ce que** le capteur est constitué d'une feuille métallique parallèle à la surface (2a) du béton et dont l'épaisseur est comprise entre 20 et 500 micromètres.

7. Procédé selon l'une des revendications 1 à 6, **caractérisé en ce que** l'élément métallique comprend un état de surface adapté de manière à contrôler la localisation de l'initiation de la corrosion et, le cas échéant, la propagation de celle-ci.

8. Procédé selon l'une des revendications 1 à 7, **caractérisé en ce que** les dimensions de l'élément métallique et la durée de l'excitation magnétique sont choisies pour conduire à une élévation de sa température d'au moins 2°C.

9. Procédé de fabrication d'un ouvrage en béton, **caractérisé en ce que** l'on met en place dans le béton au moins un capteur comprenant un élément métallique à une profondeur déterminée entre la surface du béton et le premier lit d'armatures et **en ce que** l'on met en oeuvre sur ledit ouvrage le procédé de contrôle de la corrosion selon l'une des revendications 1 à 8.

## Patentansprüche

1. Verfahren zum Prüfen der Korrosion eines Bauwerks aus Beton, wobei das Bauwerk wenigstens einen Sensor umfasst, der ein metallisches Element (1, 1', 1") umfasst, und dies in einer festgelegten Tiefe zwischen der Oberfläche des Betons und der ersten Bewehrungslage, wobei das Verfahren **dadurch gekennzeichnet ist, dass** es den Sensor betreffend die Messung der Korrosion des metallischen Elements durch den Einsatz folgender Phasen umfasst:
• Erhitzen durch Induktion des metallischen Elements (1, 1', 1") durch die Anwendung einer magnetischen Anregung oberhalb der Oberfläche (2a) des Bauwerks (2),
• Erstellen wenigstens eines Thermografiebildes der Oberfläche (2a) des Bauwerks (2),
• Ableiten, ausgehend vom Thermografiebild, des Korrosionsgrads des metallischen Elements (1, 1', 1").

2. Verfahren nach Anspruch 1, **dadurch gekennzeichnet, dass** nach dem Abschalten der magnetischen Anregung mehrere aufeinanderfolgende Thermografiebilder erstellt werden, derart, dass die Temperaturveränderung des metallischen Elements (1, 1', 1 ") im Zeitverlauf gemessen wird.

3. Verfahren nach Anspruch 1, **dadurch gekennzeichnet, dass** nach dem Abschalten der magnetischen Anregung mehrere aufeinanderfolgende Thermografiebilder erstellt werden, derart, dass wenigstens zwei auf den Bildern sichtbare metallische Elemente (1, 1', 1 ") durch ihre Temperatur unterschieden werden.

4. Verfahren nach einem der Ansprüche 1 bis 3, **dadurch gekennzeichnet, dass** die magnetische Anregung durch ein Magnetfeld mit einer Stärke von mehr als 0,1 mT, einer Frequenz zwischen 10 kHz und 1 MHz und einem Abstand zwischen 0,1 cm und 10 cm zur Oberfläche (2a) des Bauwerks (2) erzeugt wird.

5. Verfahren nach einem der Ansprüche 1 bis 4, **dadurch gekennzeichnet, dass** der Sensor in einer Tiefe zwischen 0,1 und 10 cm unter der Oberfläche des Betons angeordnet wird.

6. Verfahren nach einem der Ansprüche 1 bis 5, **dadurch gekennzeichnet, dass** der Sensor aus einer Metallfolie gebildet wird, die parallel zur Oberfläche (2a) des Betons angeordnet ist und deren Dicke zwischen 20 und 500 Mikrometer liegt.

7. Verfahren nach einem der Ansprüche 1 bis 6, **dadurch gekennzeichnet, dass** das metallische Element einen Oberflächenzustand umfasst, der dafür eingerichtet ist, die Lokalisierung der Auslösung der Korrosion und gegebenenfalls deren Ausbreitung zu steuern.

8. Verfahren nach einem der Ansprüche 1 bis 7, **dadurch gekennzeichnet, dass** die Abmessungen des metallischen Elements und die Dauer der magnetischen Anregung so gewählt sind, dass sie zu einer Anhebung seiner Temperatur von wenigstens 2°C führen.

9. Verfahren zur Herstellung eines Bauwerks aus Beton, **dadurch gekennzeichnet, dass** in den Beton wenigstens ein Sensor eingebaut wird, der ein metallisches Element in einer festgelegten Tiefe zwischen der Betonoberfläche und der ersten Bewehrungsschicht umfasst, und dadurch, dass an dem Bauwerk das Verfahren zum Prüfen der Korrosion nach einem der Ansprüche 1 bis 8 eingesetzt wird.

## Claims

1. Process for controlling the corrosion of a concrete structure, in which said structure includes at least one sensor comprising a metal element (1, 1', 1") located at a determined depth between the surface of the concrete and the first reinforcement bed, said process being **characterized in that** it includes, for said sensor, the measurement of the corrosion of said metal element by implementing:
- the induction heating of said metal element (1, 1', 1"), by applying magnetic excitation above the surface (2a) of the structure (2),
- the production of at least one thermographic image of the surface (2a) of the structure (2),
- and the deduction, on the basis of the thermographic image, of the degree of corrosion of the metal element (1, 1', 1").

2. Process according to claim 1, **characterized in that** a plurality of successive thermographic images are obtained after the magnetic excitation is stopped, so as to measure the change over time in the temperature of said metal element (1, 1', 1").

3. Process according to claim 1, **characterized in that** a plurality of successive thermographic images are obtained after the magnetic excitation is stopped, so as to distinguish, by their temperature, at least two metal elements (1, 1', 1") visible in said images.

4. Process according to one of claims 1 to 3, **characterized in that** the magnetic excitation is applied with a magnetic field having an intensity greater than 0.1mT, a frequency of between 10kHz and 1MHz and at a distance of between 0.1cm and 10cm with respect to the surface (2a) of the structure (2).

5. Process according to one of claims 1 to 4, **characterized in that** the sensor is arranged at a depth of between 0.1 and 10cm below the surface of the concrete.

6. Process according to one of claims 1 to 5, **characterized in that** the sensor consists of a metal sheet parallel to the surface (2a) of the concrete and of which the thickness is between 20 and 500 micrometers.

7. Process according to one of claims 1 to 6, **characterized in that** the metal element includes a surface state suitable for controlling the localization of the initiation of the corrosion and, as the case may be, the propagation of same.

8. Process according to one of claims 1 to 7, **characterized in that** the dimensions of the metal element and the duration of the magnetic excitement are chosen so as to lead to an increase in its temperature by at least 2°C.

9. Process for producing a concrete structure, **characterized in that** at least one sensor including a metal element is placed in the concrete at a determined depth between the surface of the concrete and the first reinforcement bed and **in that** the corrosion inspection process according to one of claims 1 to 8 is implemented on said structure.
